# EUROPEAN PATENT APPLICATION

(11) **EP 4 844 406 A2**
(43) Date of publication of application: **25.11.2026**
(21) Application number: 26209855.1
(22) Date of filing: 05.01.2010
(51) Int. Cl.: G01N 33/50

(54) **HIGH-THROUGHPUT PRODUCTION OF INGESTIBLE EVENT MARKERS**

(30) Priority: 06.01.2009 US 142849 P
(62) Document number(s) of the earlier application(s) in accordance with Art. 76 EPC: 10729424.1 / 2 385 828
(71) Applicant: Otsuka Pharmaceutical Co., Ltd., Tokyo 101-8535 (JP)
(72) Inventor: MOON, Gregory, California, 94563 (US); HAFEZI, Hooman, California, 94065 (US); ZDEBLICK, Mark, California, 94028 (US); AU-YEUNG, Kityee, California, 94107 (US); DUCK, Robert, California, 94109 (US); HOLEN, Maria, California, 95054 (US); ROBERTSON, Timothy, California, 94002 (US); SNYDER, Eric, California, 94080 (US); FOLENA, Mauro, California, 95033 (US); COSTELLO, Benedict, California, 94703 (US); RAO, Pratish, California, 94002 (US)
(74) Representative: Murgitroyd & Company

(57) **Abstract**

High-throughput ingestible event marker manufacturing systems are provided. The systems include an assembly unit configured to stably associate an ingestible event marker with a carrier to produce a product. Also provided are manufacturing methods to produce such products which include assembling an ingestible event marker with an assembly unit configured to stably associate an ingestible event marker with a carrier.

## Description

### CRoss-REFERENCE To RELATED APPLICATIONS

This application claims the benefit of U.S. Provisional Patent Application Number 61/142,849, filed January 6, 2009 and titled "High-Throughput Production of Ingestible Event Markers", incorporated by reference for all purposes in the Present Application.

### INTRODUCTION

Ingestible event markers include consumable devices which emit a detectable signal upon contact with a target internal physiological site. While configurations for ingestible event markers may vary, in certain instances ingestible event markers include one or more ingestible event marker that are stably associated with a carrier, such as a tablet or capsule. The ingestible event marker may vary, and in certain instances includes an integrated circuit component and two dissimilar materials, e.g., two electrodes. Further components include, for example, a current path extender and/or various other components which may, in certain instances, may be associated with a framework. When the ingestible event marker (sometimes referred to herein in certain aspects as an "ingestible event marker identifier" or "identifier"), contacts fluid at an internal target site, such as stomach fluid, a power source is completed that provides power to the integrated circuit component to provide a communication.

Ingestible event markers hold great promise for use in a variety of different applications. One application of interest is monitoring how a patient adheres to a prescribed pharmaceutical therapeutic regimen. In these applications, ingestible event markers are the pharmaceutical dosages of the therapeutic regimen, where the carrier component of the marker may include an active pharmaceutical ingredient of interest or be a placebo, as desired. By monitoring for communications, e.g., a current path associated with the ingestible event marker, accurate information regarding patient adherence with a prescribed pharmaceutical therapeutic regimen may be obtained. Patient adherence data obtained with ingestible event markers holds great promise, both with patients who have been prescribed approved pharmaceuticals and with patients who are participating in clinical trials.

Ingestible event markers also hold promise in protocols that do not involve administration of a pharmaceutically active agent. For example, ingestible event markers may be used to monitor an occurrence of interest, such as a mealtime, a symptom, etc. As such, applications in which ingestible event markers may find use include dieting, monitoring patients for physiological symptoms of interest, and the like.

### SUMMARY

High-throughput ingestible event marker manufacturing systems are provided. The systems include an assembly unit configured to stably associate an ingestible event marker with a carrier to produce a product. Also provided are manufacturing methods used to produce the products.

### BRIEF DESCRIPTION OF THE FIGURES

FIGS. 1A to 1B provide various views of an ingestible event marker with a signal amplification element according to an aspect of the invention. FIGS. 1C to 1G provide views of ingestible event marker that include protective coatings
FIG. 2 provides a schematic of tablet assembly unit according to one aspect of the invention.
FIG. 3 provides a schematic of tablet assembly unit according to another aspect of the invention.
FIG. 4 provides a schematic of tablet assembly unit configured to employ a tablet precursor powder according to an aspect of the invention.
FIGS. 5A to 5C provide views of tablet configurations produced from pre-made tablet precursors in accordance with the invention.
FIG. 6 provides a schematic of tablet assembly unit that includes a punch according to an aspect of the invention.
FIG. 7 provides a cross-sectional view of an ingestible event marker that includes an ingestible event marker present in a donut-shaped tablet.
FIG. 8 provides a schematic of capsule assembly unit according to an aspect of the invention.
FIG. 9A provides a view of an ingestible event marker sandwiched between two conformal capsule halves.
FIG. 9B provides a view of an ingestible event marker stably associated with an external surface of a capsule by an elastic band.
FIG. 10 provides views of various ingestible event marker configurations.
FIG. 11 provides of a diagram of various aspects of a tablet production process.
FIG. 12 provides a process flow diagram for producing an ingestible event marker having an ingestible event marker on a surface of a pre-made tablet.
FIG. 13 provides a process flow diagram for producing an ingestible event marker having an ingestible event marker inside of a tablet.
FIG. 14 provides a process flow diagram for producing an ingestible event marker having an ingestible event marker inside of a capsule.
FIG. 15 provides a schematic of an ingestible event marker production system according to an aspect of the invention.
FIG. 16 provides a process flow diagram for of a system for producing an ingestible event marker having ingestible event marker inspection functionality.
FIG. 17 provides a process flow diagram for of a system for producing an ingestible event marker having ingestible event marker programming functionality.

### DETAILED DESCRIPTION

High-throughput ingestible event marker manufacturing systems are provided. The systems include an assembly unit configured to stably associate an ingestible event marker (also known as an ionic emission module or identifier and referred to as an "IEM") with a carrier to produce a product capable of producing a unique current signature. Also provided are methods of using systems of the invention to produce ingestible event markers.

As summarized above, automated systems for high-throughput production of ingestible event markers are provided. By "high-throughput" is meant that the systems are configured to produce 100 or more ingestible event markers per hour, such as 500 or more ingestible event markers per hour, including 1000 or more ingestible event markers per hour, and in certain aspects produce 50,000 or more, 100,000 or more, 250,000 or more, 400,000 or more ingestible event markers per hour. As the systems are automated, at least some unit, i.e., sub-portion, of the system operates by automation, as opposed to operating under human control. Accordingly, one or more of the units of the system are under automatic, as opposed to human, operation or control. The system may employ any convenient type of automatic control, such as electronic (for example as embodied by computer) control. Aspects of systems of the invention include the production of ingestible event markers in which association of the ingestible event marker has no impact on drug product effectiveness, such that pharmacological properties are within required parameters. In addition, product ingestible event markers produced by systems of the invention enjoy good patient acceptance, in that they are convenient to use and attractive to patients. High throughput systems of the invention include systems of commercial scale, which provide for low-cost production of ingestible event markers. Ingestible event markers produced by systems of the invention exhibit desirable performance in the form of high detection rate and yield. Where desired, systems of the invention include integrated ingestible event marker inspection and programming functionality. Production ingestible event markers exhibit suitable shelf-life.

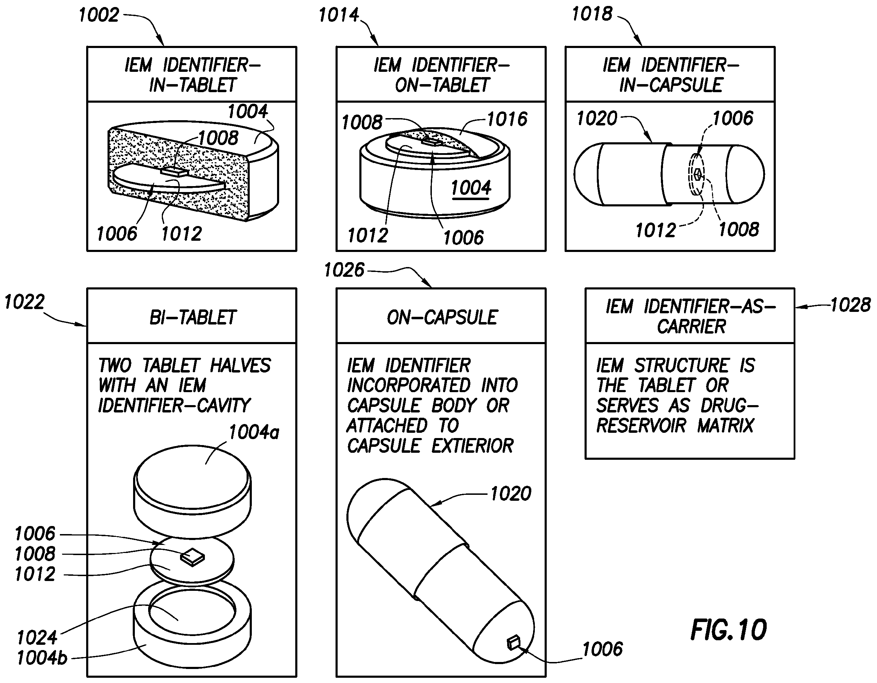

Aspects of systems of the invention include an assembly unit configured to stably associate one or more ingestible event marker with a carrier, such as a tablet or capsule, to produce an ingestible event marker. Ingestible event markers may have a variety of different configurations. Configurations of interest include, but are not limited to, those shown in FIG.10, which include various configurations.

For example, in "IEM Identifier-in-Tablet" 1002, an IEM 1006 having a unit 1008, e.g., two dissimilar materials and a control device, and a current path extender ("skirt") 1012 is present inside of a tablet 1004, e.g., by incorporation during tablet pressing or placement in a cavity provided by two tablet halves.

In "IEM Identifier-On-Tablet" 1014, an IEM 1006 having a unit 1008, e.g., two dissimilar materials and a control device, and a current path extender ("skirt") 1012 is communicably associated with a tablet 1004. A coating 1016, shown in partial form, partially or wholly covers the IEM 1005 and may cover at least a portion of the carrier, e.g., tablet 1004.

In "IEM Identifier-As-Carrier" 1018, an IEM 1006 having a unit 1008, e.g., two dissimilar materials and a control device, and a current path extender ("skirt") 1012 is communicably associated, e.g., inserted into, a capsule 1020.

In "Bi-Tablet" 1022, an IEM 1006 having a unit 1008, e.g., two dissimilar materials and a control device, and a current path extender ("skirt") 1012 is communicably associated, e.g., disposed within two tablet-halves 1004a and 1004b, respectively.

In "On-Capsule" 1026, an IEM 1006 having a unit 1008 is communicably associated, e.g., attached to an exterior portion of capsule 1020.

In "IEM Identifier-As-Carrier" 1028, an IEM 1006 structure is the tablet or serves as a drug-reservoir matrix. To illustrate, an ingestible event marker includes an integrated carrier structure, where a current path extender ("skirt") serves as drug matrix. By "stably associate" is meant that the one or more markers are physically associated with the carrier component of the ingestible event marker prior to ingestion. A given ingestible event marker may be associated with a carrier, such as a tablet or capsule, using a variety of different approaches. For example, physiological acceptable adhesives, such as thermoset, solvent evaporation, or other types of adhesives may be employed. Alternatively, welding elements, such as tabs or other structures, which can be melted with a high energy stimulus (such as a laser, ultrasonic source, etc.), may be employed to stably associate the ingestible event marker with a carrier. Alternatively, one or more components of the ingestible event marker may be manufactured on a carrier or carrier precursor thereof (such as by use of pulse-jet protocols described in greater detail below) in a manner that stably associates the ingestible event marker with the carrier. Also of interest is the use of ingestible event marker that include structures (such as elastic bands, press-fit structures, etc.) configured to mechanically interact with a carrier to provide the desired stable association of the ingestible event marker with the carrier. Such structures are elements that mechanically provide for stable association of the ingestible event marker with the pre-made carrier.

Ingestible event markers of interest are structures that produce a unique current signature that indicates occurrence of an event. In one example, the ingestible event marker emits a detectable current signature upon contact of the ingestible event marker with a target physiological location (or locations). In another example, the ingestible event marker includes dissimilar materials positioned on a framework such that when a conducting fluid, e.g., stomach acid, comes into contact with the dissimilar materials, a voltage potential difference is created. The voltage potential difference, and hence the voltage, is used to power up control logic that is positioned within the framework. The control logic produces the unique current signature. Other examples of components that may be included are: logic and/or memory elements; effectors; a non-conductive element to extend the current path (sometimes referred to herein as a "skirt); a signal transmission element; and a passive element, such as a resistor or inductor.

The ingestible event marker may vary depending on the particular aspect and intended application of the composition, as long as they are activated (turned on) upon contact with a target physiological location, such as the stomach or small intestine. As such, an ingestible event marker may be a structure that emits a signal when activated at a target site, for example when it contacts a target body site. The ingestible event marker may be any component or device that is capable of providing a detectable signal following activation. Ingestible event markers according to aspects of the invention include a signal generation component. The ingestible event marker may be configured to emit a signal once the composition comes into contact with a physiological target site. Depending on the aspect, the target physiological site or location may vary, where representative target physiological sites of interest include, but are not limited to: a location in the gastrointestinal tract, such as the mouth, esophagus, stomach, small intestine, large intestine, etc. Ingestible event marker may be configured to be activated upon contact with fluid at the target site, e.g., stomach fluid, regardless of the particular composition of the target site. Where desired, the ingestible event marker may be configured to be activated by interrogation, following contact of the composition with a target physiological site. The ingestible event marker may be configured to be activated at a target site, where the target site is reached after a specified period of time.

Depending on the needs of a particular application, the signal obtained from the ingestible event marker may be a generic signal, such that the signal is a signal that merely identifies that the composition has contacted the target site. Alternatively, the signal may be a unique current signature, such as a signal which in some way uniquely identifies that a particular ingestible event marker from a group or plurality of different ingestible event markers, for example a batch of ingestible event markers, has contacted a target physiological site. As such, the ingestible event marker may be one that emits a signal that cannot be distinguished from the signal emitted by the ingestible event marker of any other ingestible event marker member of a batch from which the ingestible event markers are obtained. Alternatively, each ingestible event marker member of a batch of ingestible event markers may have an ingestible event marker that emits a unique current signature, at least with respect to all of the other ingestible event markers of the ingestible event marker members of the batch. The ingestible event marker may emit a unique current signature that is a universally unique current signature (where such a signal may be analogous to a human fingerprint which is distinct from any other fingerprint of any other individual and therefore uniquely identifies an individual on a universal level). The signal may either directly convey information about a given event, or provide an identifying code, which may be used to retrieve information about the event from a database, such as a database linking identifying codes with compositions.

The ingestible event marker may generate a variety of different types of signals, including but not limited to: RF signals, magnetic signals, conductive (near field) signals, acoustic signals, etc. The transmission time of the ingestible event marker may vary, where in certain instances the transmission time may range from 0.1 µsec to 48 hours or longer, such as from 0.1 µsec to 24 hours or longer, such as from 0.1 µsec to 4 hours or longer, such as from 1 sec to 4 hours, including from 1 minute to 10 minutes. Depending on the given aspect, the ingestible event marker may transmit a given signal once. Alternatively, the ingestible event marker may be configured transmit a signal with the same information (identical signals), two or more times, where the collection of discrete identical signals may be collectively referred to as a redundant signal.

The ingestible event marker may vary depending on the particular aspect and intended application of the composition so long as they are activated upon contact with a target physiological location, such as the stomach. Ingestible event marker may include an activation component, such as a partial power source that is completed by stomach acid, and a transmission element. Examples of different types of ingestible event marker of interest include, but are not limited to, those ingestible event marker described in PCT application serial no. PCT/US2006/016370 published as WO/2006/116718; PCT application serial no. PCT/US2007/082563 published as WO/2008/052136; PCT application serial no. PCT/US2007/024225 published as WO/2008/063626; PCT application serial no. PCT/US2007/022257 published as WO/2008/066617; PCT application serial no. PCT/US2008/052845 published as WO/2008/095183; PCT application serial no. PCT/US2008/053999 published as WO/2008/101107; PCT application serial no. PCT/US2008/056296 published as WO/2008/112577; PCT application serial no. PCT/US2008/056299 published as WO/2008/112578; and PCT application serial no. PCT/US2008/077753; the disclosures of which are herein incorporated by reference.

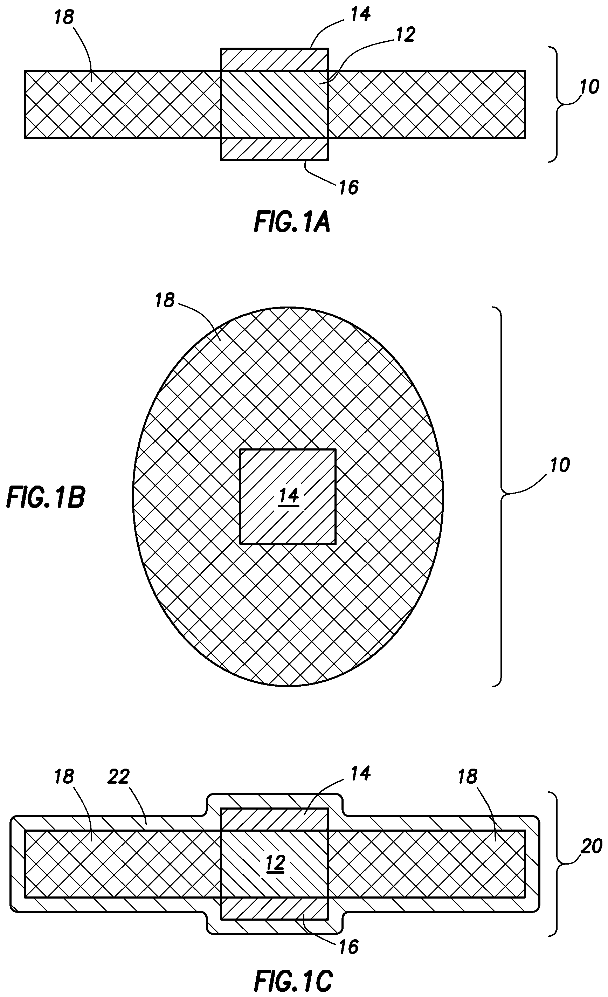

An example of an ingestible event marker of interest is depicted in FIGS. 1A and 1B. The ingestible event marker shown in FIGS. 1A and 1B includes an integrated circuit component as well as upper and lower electrodes, where the upper and lower electrodes are configured such that upon contact with stomach fluid current runs through the integrated circuit to cause one or more functional blocks in the circuit to emit a detectable signal. The marker shown in FIGS. 1A and 1B includes a virtual dipole signal amplification element, as reviewed in greater detail in PCT application serial no. PCT/US20008/077753, the disclosure of which is herein incorporated by reference.

FIG. 1A provides a view of an aspect of an ingestible event marker which has a signal amplification element that extends beyond the outer edges of the upper and lower electrodes (which also serve as signal transmission elements) to provide a virtual dipole having a length that is longer than the actual dipole between the signal transmission elements. As shown in FIG. 1A, ingestible event marker 10 includes integrated circuit component 12, having an upper electrode 14 and a lower electrode 16 (which may comprise two distinct material layers). Also shown is disc-shaped signal amplification element or non-conductive current path extender ("skirt") 18

FIG. 1B provides an overhead view of the ingestible event marker of FIG. 1A, showing the disc shape of upper electrode 14 and the positioning of the upper electrode in the center of disc-shaped signal amplification element 18. The distance that the edge of the signal amplification element may extend beyond the edge of electrodes may vary, and in certain instances is 0.05 mm or more, e.g., 0.1 mm or more, including 1.0 mm or more, such as 5.0 mm or more and including 10 mm or more, where the distance may not exceed 100 mm in some instances.

As can be seen in the aspect depicted in FIGS. 1A to 1B, the upper and lower electrodes are planar electrodes, where these electrodes may have any convenient shape, such as square, disc, triangular, oval, irregular, etc. The disc-shaped signal amplification element 18 is a planar disc structure, where the edge of the signal amplification element extends beyond the edge of the planar upper and lower electrodes. In the depicted ingestible event marker, the radius of the signal amplification element is longer than the radius of the upper and lower electrodes, for example by 1 mm or more, such as by 10 mm or more.

Ingestible event markers produced by systems of the invention may be configured in a variety of different formats. Formats of interest include, but are not limited to, tablets and capsules. The automated ingestible event marker assembly units may vary depending on the particular format of the ingestible event marker. Depending on the particular configuration, a given assembly unit may include a number of elements configured to move ingestible event marker and carrier components from a first location, such as a source (for example a hopper) to a second location, such as an assembly site. Elements configured to move ingestible event marker components (such as ingestible event marker and carriers or carrier precursors thereof) from one position to another within the system include, but are not limited to: feeders, air guns, rollers and drums, conveyers, robotic positions configured to pick up and place small objects at specific locations, tape punchers, etc.

Ingestible event marker may include one or more processing specific components that are used by a given system during manufacture of ingestible event markers. Examples of processing specific components include orientation components, handling components, protection components, etc.

In some instances, ingestible event marker may include a processing specific component that serves to position the ingestible event marker relative to a reference point, e.g., a carrier component, a tablet press, etc., during manufacture of the ingestible event markers by the assembly unit. For example, ingestible event marker may include a weight component which ensures the ingestible event marker always faces a certain direction (such as circuit side up) relative to a carrier component with which it may be associated during processing. In some instances this weight component is a small tablet that is adhered to the ingestible event marker, for example with a physiologically acceptable adhesive.

In some instances, ingestible event marker of the invention may include processing component that serves to protect the ingestible event marker during manufacture of the ingestible event markers by the system. An example of such a processing component is a coating, which coating may cover one or more surfaces of the ingestible event marker or all of the surfaces of the ingestible event marker, such that the ingestible event marker is enveloped by the coating. The protection function of the coating can vary. For example, the coating can be configured to protect the ingestible event marker or components thereof, such as the integrated circuit component, the electrode components, etc., during processing, during storage, or even during ingestion. For instance, one may not desire the circuitry to be exposed to the body fluids after it is ingested. In such instances, it may be desirable to only have the incomplete battery and transmit antennas be exposed to body fluids, with the rest of the circuitry being protected. In such instances, a coating on top of the circuitry component that is ingestible but does not dissolve until the device has finished its signal transmission may be provided. Also of interest are coatings that are designed to protect the ingestible circuitry component during storage, but that dissolve immediately during use. For example, coatings that dissolve upon contact with an aqueous fluid, such as stomach fluid, may be employed. Also of interest are protective processing coatings that are employed to allow the use of processing steps that would otherwise damage certain components of the device. In such instances, a protective coating on the ingestible event marker that prevents mechanical or liquid contact with the ingestible event marker during processing can be employed. Coatings of interest include pliable coatings that protect the ingestible event marker from forces experienced during ingestible event marker manufacture, such as tablet compression forces. Coatings of interest also include environmentally sensitive coatings, such as pH sensitive coatings that protect the ingestible event marker from a liquid present in a capsule but then dissolve once the ingestible event marker contacts stomach fluid. Examples of such coatings are liquid protective coatings that prevent the circuit component of the ingestible event marker from being activated by liquid of the carrier component of the ingestible event marker. Another purpose of the coatings may be to control the activation of the device. For example, an edible coating that covers the electrodes and takes a certain period of time, e.g., five minutes, to dissolve upon contact with stomach fluid may be employed. The coating can also be an environmentally sensitive coating, e.g., a temperature or pH sensitive coating, or other chemically sensitive coating that provides for dissolution in a controlled fashion and allows one to activate the device when desired. Coatings that survive the stomach but dissolve in the intestine are also of interest, e.g., where one desires to delay activation until the device leaves the stomach. An example of such a coating is a polymer that is insoluble at low pH, but becomes soluble at a higher pH. Coatings of interest further include hot-melt coatings and sugar coatings.

Of interest in certain instances are hydrogel coatings. Hydrogel coatings are polymeric coatings made up of one or more different types of non-water soluble polymers, where the coatings absorb water upon contact with an aqueous medium to produce a hydrated gel-structure that has a high water content, such as 90% or more w/w, including 95% or more w/w, such as 99% or more w/w. Any physiologically acceptable hydrogel composition may be employed as a coating, where hydrogel compositions of interest may include one or more of the following polymers: polyethylene oxides, acetates, etc. In the hydrated gel structure state, the coating may be pliable, and thereby protect the ingestible event marker during processing, for example by cushioning the ingestible event marker during tablet pressing, etc. In some instances, the hydrogel coating may include one or more agents which provide for a controlled environment (for example in terms of conductivity) when the ingestible event marker reaches the target physiological site. Agents of interest include, but are not limited to: salts of physiologically acceptable electrolytes, such as but not limited to: sodium ion, chloride ion, potassium ion and calcium ion, magnesium ion, etc. Specific physiologically compatible salts of interest include, but are not limited to: KCI, NaCl, MgCl₂, and the like.

Coatings may take a variety of different configurations, such as layers, snap-fit pre-made capsule components, etc. When present, coatings may cover only a portion of the ingestible event marker or envelope the entire. The coating may be uniform in terms of thickness. An example of an ingestible event marker that is enveloped in a coating of uniform thickness is shown in FIG. 1C. In FIG. 1C, ingestible event marker 20 includes integrated circuit component 12, having an upper electrode 14 (which may comprise two distinct material layers) and a lower electrode 16, as well disc-shaped signal amplification element 18. Also shown is uniform coating 22.

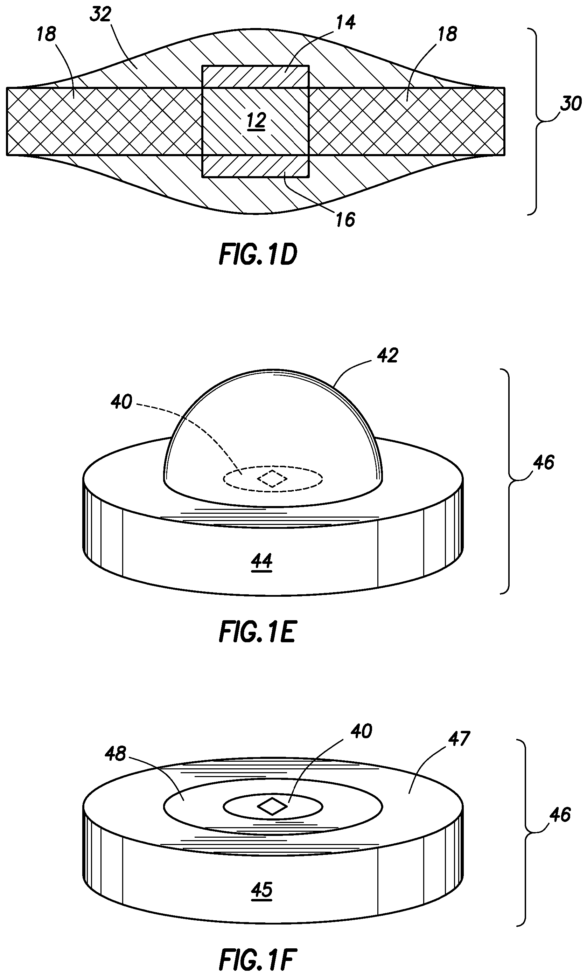

Alternatively, the coating may be non-uniform, e.g., where the coating is thicker towards the center of the ingestible event marker as opposed to the edges of the ingestible event marker. An example of an ingestible event marker that is enveloped in a coating of non-uniform thickness is shown in FIG. 1D. In FIG. 1D, ingestible event marker 30 includes integrated circuit component 12, having an upper electrode 14 (which may comprise two distinct material layers) and a lower electrode 16, as well disc-shaped signal amplification element 18. Also shown is non-uniform coating 32, which non-uniform coating 32 is thicker above and below the electrode components 14 and 16 and thinner towards the outer edge of the signal amplification element 18.

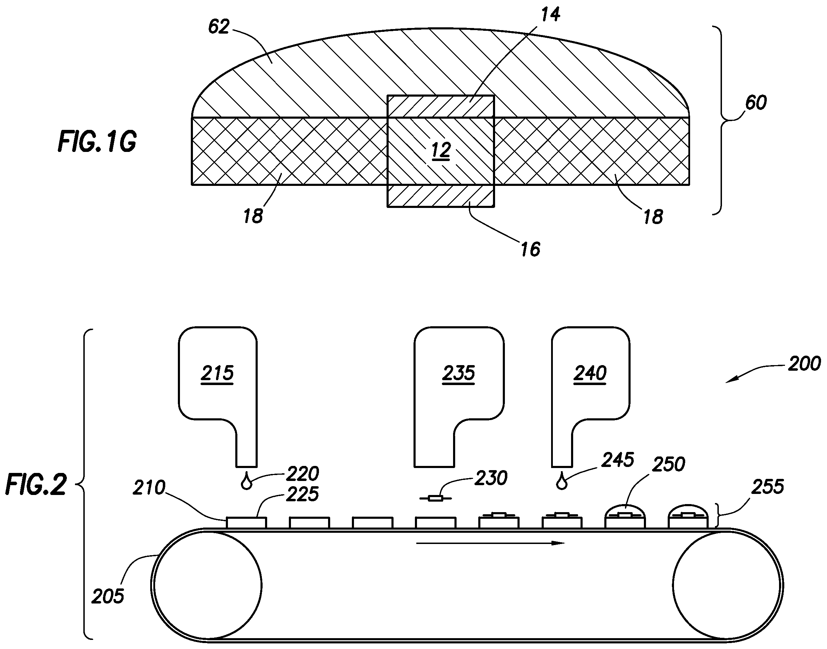

In some instances, the coating covers only a portion of the ingestible event marker. FIG. 1E provides a view of the ingestible event marker identifier 40 covered on one surface by a coating in the form of a protective cap 42. Also shown in FIG. 1E is carrier 44 in the form of a tablet, where the carrier 44 together with the ingestible event marker identifier 40 and protective cap 42 make up an ingestible event marker 46. FIG. 1G shows a variation of an ingestible event marker 60 which may be employed in systems of the invention. Ingestible event marker 60 is made up of integrated circuit component 12, upper and lower electrodes 14 and 16 and signal amplification element 18. In the view shown in FIG. 1G, protective cap 62 is pre-affixed to the upper surface of the ingestible event marker before the ingestible event marker is adhered to a tablet carrier. In an alternative configuration shown in FIG. 1F, protective cap 42 from FIG. 1E has been replaced by protective sheet 48. Where desired, the protective sheet 48 may fit with the upper surface 47 of a tablet component 45 to be level with the upper surface 47 of the tablet component 45. The tablet component 45 may define a cavity (not shown) for receiving the ingestible event marker 40 such that when the protective sheet 48 is fitted into the cavity it is approximately level with the upper surface 47.

Any coating associated with an ingestible event marker may be opaque in order to prevent the end-user from seeing the ingestible event marker associated with carrier of the ingestible event marker. Alternatively, the coating may be transparent so as to provide ready visualization of the ingestible event marker that is stably associated with the carrier.

With respect to fabrication of such coatings, the coatings may be associated with the ingestible event marker using any convenient protocol, such as by use of a fluidized bed, deposition as well as instances where the coating is pre-made and then fit over the ingestible event marker, such as a cap that is glued onto a portion of the ingestible event marker or capsule components that are press-fit onto a composite structure of an ingestible event marker and a carrier, such as a tablet. In some instances, the assembly unit itself may be configured to associate a coating with the ingestible event marker, such as by dispensing a precursor coating fluid onto an ingestible event marker, where the precursor coating fluid cures into the desired coating.

The ingestible event marker may include one or more features that allow the markers to be readily manipulated by the system during manufacture. For example, signal amplification elements of ingestible event marker may be configured to be employed as handles (for example by having a rigid component that is configured to be grabbed by a manipulator of the assembly unit of the system).

Assembly units of systems of the invention may vary greatly depending on the manufacturing protocol employed by the system to produce ingestible event markers from ingestible event marker and carriers or precursors thereof. For example, assembly units may be units configured to stably associate an ingestible event marker with a pre-made carrier, such as a pre-made tablet or capsule. Pre-made carriers include provided, e.g., pre-assembled, manufactured, and/or previously configured units, etc., carriers. One example is a fully-formed capsule or tablet. Alternatively, assembly units may be units configured to stably associate an ingestible event marker with one or more carrier component precursors, such as pre-made tablet halves, tablet precursor powders, capsule halves, etc. Carrier component precursors include one or more components, sub-components, and combinations thereof of carriers. One such example is powder used in the production of a table. Another such example is one portion, e.g., one-half of, a capsule shell or housing. Yet another example is a printed ingestible event marker component such as a portion of circuitry associated with an ingestible event marker. In yet other aspects, assembly units may be configured to stably associate an ingestible event marker with a carrier during packaging, where the assembly unit is integrated with the packaging unit of the system.

Assembly units of systems of the invention may be configured to employ pre-made ingestible event marker, such as ingestible event marker that have been produced by another unit of the system or ingestible event marker that are obtained from a distinct source, such as another distinct manufacturing system.

Alternatively, assembly units of systems of the invention may be configured to produce one or more components of an ingestible event marker, such as a signal amplification element, circuitry components, electrode components, etc., on a portion (such as a surface) of a pre-made carrier component or precursor thereof. As such, assembly units may be configured to produce one or more components of the ingestible event marker on a surface of a pre-made carrier. In some systems of the invention, pulse-jet devices (also known as ink-jet devices, which may include piezoelectric and thermal pulse-jet dispensers) are employed to deposit one or more components of an ingestible event marker onto a surface of a carrier in an assembly unit of the system. For example, a pre-made tablet having a signal-amplification element cavity shaped to serve as mold on its upper surface may be positioned in receiving relationship relative to a pulse-jet device that deposits a volume of signal-amplification element material into the cavity to produce a signal amplification element on a surface of the tablet. Next, an integrated circuit component may be positioned in the center of the signal-amplification element to produce an ingestible event marker having an ingestible event marker adhered to an upper surface of the tablet carrier. Where desired, a coating layer may then be deposited using a pulse-jet deposition device onto the upper surface of the tablet and the ingestible event marker, for example to produce an ingestible event marker 46 as shown in FIG. 1E. In some systems, pulse-jet technology may be employed to produce other components of the ingestible event marker, such as electrodes, conductive traces, other circuitry components, etc.

As summarized above, ingestible event markers produced by systems of the invention include one or more ingestible event marker identifiers stably associated with a carrier. The carrier may have a variety of different configurations, where examples of carriers include, but are not limited to, tablets, capsules, multi-dose tapes that can be separated at the time of use to obtain an individual ingestible event marker, etc. Depending on the particular application for which the ingestible event marker is being fabricated, the carrier may or may not include a pharmaceutically active agent. As such, the carrier component that is associated with the ingestible event marker in the ingestible event marker may not include a pharmaceutically active agent. In yet other aspects, the carrier component may include one or more pharmaceutically active agents As used herein, the term "active agent" includes any compound that produces a physiological result, for example a beneficial or useful result, upon contact with a living organism, such as a human. Active agents are distinguishable from vehicle components such as fillers, binders, coloring agents, etc. The active agent may be any molecule that is capable of modulating a biological process in a living subject. In some instances, the active agent may be a substance used in the diagnosis, treatment, or prevention of a disease or as a component of a medication. Broad categories of active agents of interest include, but are not limited to: cardiovascular agents; pain-relief agents, e.g., analgesics, anesthetics, anti-inflammatory agents, etc.; nerve-acting agents; chemotherapeutic (such as anti-neoplastic) agents; etc. Active agents of interest are further disclosed in PCT Application Serial No. US2006/016370 published as WO 2006/116718, the disclosure of which is herein incorporated by reference.

As indicated above, some aspects may include one or more ingestible event marker identifiers stably associated with a carrier. In some instances, two or more ingestible event markers are associated with different locations of the same carrier, such as different surfaces of the carrier, e.g., to ensure that signal is emitted in multiple directions. In such instances, the assembly unit is configured to stably associate the two or more ingestible event marker with different locations of the carrier. Ingestible event markers produced by systems of the invention may include a single ingestible event marker, or two or more ingestible event markers. The ingestible event markers may be arranged in a variety of different configurations with respect to the other components of the marker. Where the marker includes a tablet carrier, the ingestible event marker(s) may be arranged on a surface of the tablet carrier or inside of the tablet carrier. In some instances, the marker is made up of two or more ingestible event markers present in a capsule. For example, multiple ingestible event marker ingestible event markers may be present in a capsule fabricated from a material that dissolves upon contact with stomach fluid. The ingestible event marker associated with the carrier may be configured to emit a signal at different times, such as certain times during the controlled release of one or more active agents from an ingestible event marker. For example, a first ingestible event marker may be stably associated with a surface of a tablet carrier and a second ingestible event marker may be stably associated with an interior region of the tablet carrier.

Automated ingestible event marker assembly units configured for production of tablets and capsules are now reviewed in greater detail.

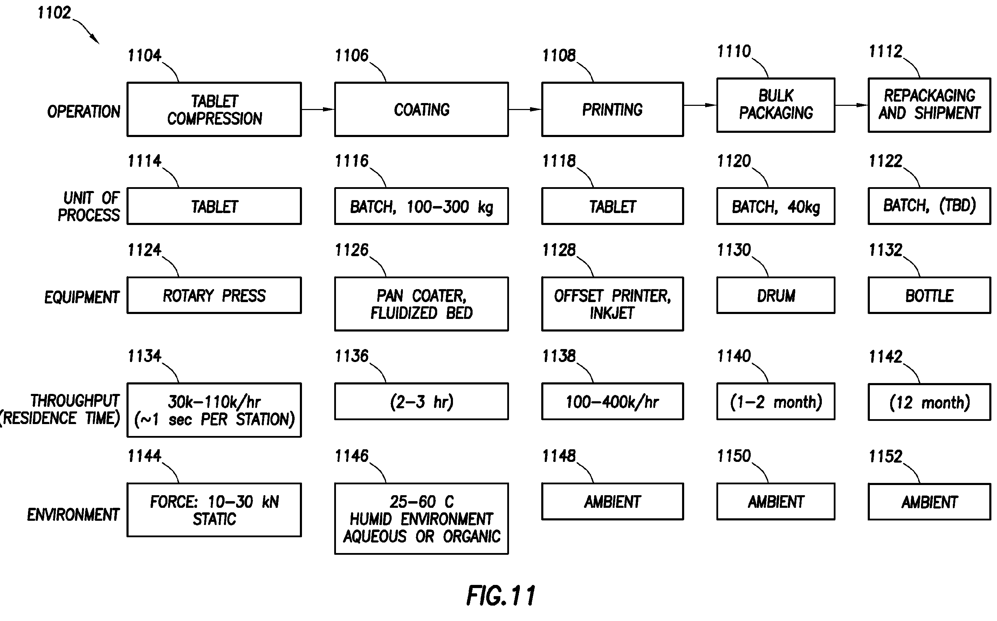

A production flow diagram 1102 of tablet assembly system of interest is provided in FIG. 11. In the process flow diagram1102, shown in FIG. 11, depending on the particular system the ingestible event marker may be readily associated with the carrier at a variety points in the production process. For example, an operation may comprise one or more steps such as tablet compression 1104, coating the tablet 1106, printing an IEM on the coated tablet 1108, bulk packaging the coated, printed tablets 1110, and repackaging the tablets. A unit of process may comprise, for example, a tablet 1114; a batch, e.g., 100-300 kg. of tablets, 1116; a tablet for printing 1118; a batch, e .g., 40 kg., for bulk packaging 1120; and a batch of bulk-packaged tablets for repackaging and shipping 1122. The equipment may comprise, for example, a rotary press 1124 for tablet compression; a pan coater, fluidized bed 1126 for coating; an offset printer inkjet 1128 for printing; a drum 1130 for bulk packaging; and a bottle 1132 for repackaging and shipment. The throughput may comprise, for example 30k - 11k/hr. (approximately 1 sec. per station) 1134 for tablet compression; 2 - 3 hours 1106 for coating; 100-400k/hr. 1138 for printing; 1 - 2 month cycle1140 for bulk packaging; and 12 month cycle 1142 for repackaging and shipment. The environment may comprise, for example, force: 10-30 kN static 1144 for tablet compression; 25-60°C humid environment, aqueous or organic 1146 for coating; ambient 1148 for printing; ambient 1150 for bulk packaging; and ambient 1152 for repackaging and shipment.

The system shown in FIG. 11 may be adapted to provide for ingestible event marker associated with a carrier and provide for high-yield at all process steps, as well as inspection at desired points and ingestible event marker tracking.

For tablets, the automated high-throughput ingestible event marker assembly unit is configured to stably associate one or more ingestible event markers with a tablet carrier component or precursors thereof (such as pre-made tablet components or powders) to produce the desired ingestible event marker having a tablet configuration. The assembly unit may be configured to produce an ingestible event marker in which the ingestible event marker is present inside of the tablet or on a surface of the tablet, as desired. Pre-made tablets may be produced from a variety of different compositions. Tablet compositions may include one or more vehicle components. Vehicle components may include one or more constituents, including but not limited to fillers, binders, disintegrants, coloring agents, etc. Vehicle components of interest are further reviewed in PCT Application Serial No. US2006/016370 published as WO 2006/116718, the disclosure of which is herein incorporated by reference. Additional disclosure of vehicle components that can be present in carriers of the invention can be found in Remington's Pharmaceutical Sciences, Mace Publishing Company, Philadelphia, Pa., 17th ed. (1985).

In some instances, the assembly unit is configured to stably associate an ingestible event marker with a pre-made tablet (for example a tablet that has been prepared by another unit of the system, such as a tablet press, or by another system entirely). For these types of assembly units, the assembly unit includes a transfer element for moving pre-formed tablets from a source to the assembly site of the system. Also present is a transfer element for moving an ingestible event marker from a source of ingestible event marker to the assembly site. These disparate transfer elements may vary, where transfer elements of interest include hoppers and feeds, robotic picking and placing devices, rollers, dispensers, etc. The assembly unit also includes a mechanism to stably associate the ingestible event marker(s) with the pre-formed tablets. Where desired, the assembly unit may include a source of adhesive and a mechanism for applying an amount of the adhesive, e.g., an adhesive dispenser, to one or both of the pre-made tablet and ingestible event marker prior to joining the ingestible event marker to the pre-made tablet. Any convenient adhesive may be employed by systems of the invention, where adhesives of interest include but are not limited to: cellulosic adhesives, pressure sensitive adhesives, etc. Assembly units configured to employ pre-made tables may also include one or more components for producing ingestible event marker on pre-made tablets, such as pulse-jet devices as described above.

With respect to the pre-made tablets employed by such systems, certain shapes may be preferred for easier handling, for example to provide for alignment when the ingestible event marker is about to be inserted into a pre-made tablet or attached to the tablet. In some instances, features, such as indentations, bumps, or holes, can be embossed into the tablet to allow the system to readily orient the ingestible event marker during its attachment or insertion, printing, testing, etc. Where desired, tablets may be pre-coated with a coating, for example with a coating as described above. In some instances, the coating may be configured to facilitate release of the ingestible event marker from the tablet upon contact of the ingestible event marker with the target physiological site, for example the stomach.

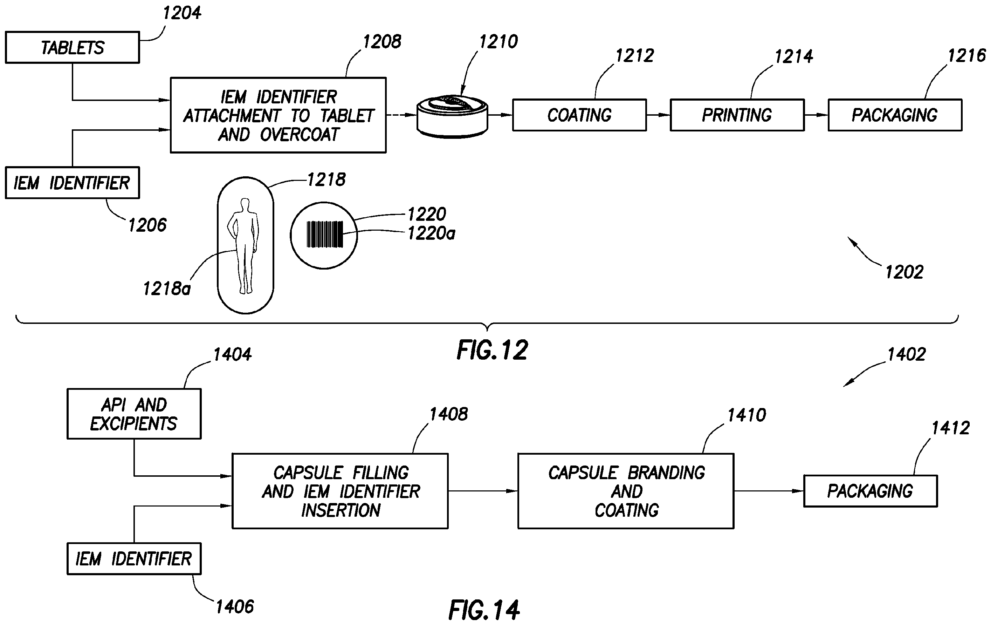

FIG. 12 provides a view of process flow diagram for a system that combines an ingestible event marker with a pre-made tablet to produce a product. For example, an IEM identifier 1206 may be attached to a tablet 1204 and over-coated at step 1208 to produce product 1210. Product 1210 may be coated at step 1212, printed at step 1214, and packaged at step 1216. Sample products are shown as capsule 1218 having a logo printed thereon and tablet 1220 having a barcode 1220a printed thereon.

An example of an assembly unit configured to stably associate an ingestible event marker with a pre-made tablet to produce an ingestible event marker is shown in FIG. 2. In FIG. 2, assembly unit 200 includes conveyor 205 which moves pre-made tablets 210 past an adhesive application device 215. Adhesive application device 215 deposits a pre-determined amount of adhesive 220 onto the upper surface 225 of each pre-made tablet 210 as the tablet 210 is moved past the adhesive application device 215. Following placement of the pre-determined amount of adhesive 220 onto the upper surface 225 of each pre-made tablet 210, an ingestible event marker 230 is deposited onto the pre-determined amount of adhesive 220 from ingestible event marker feeder 235. In the assembly unit 200 shown in FIG. 2, the ingestible event marker 230 adhered to the upper surface 225 of pre-made tablet 210 is then moved past a dispenser 240 which dispenses a volume of a precursor protector fluid 245, where the volume of the fluid 245 solidifies into a protective cap 250 that covers the ingestible event marker 230, e.g., to protect it during further processing, to hide the ingestible event marker 230, etc. As reviewed above, the protection material may be opaque, for example to hide the ingestible event marker 230 from the consumer of the product. Finished ingestible event markers 255 produced by the assembly unit 200 are then forwarded to other parts of the system, e.g., inspection units, packaging units, etc.

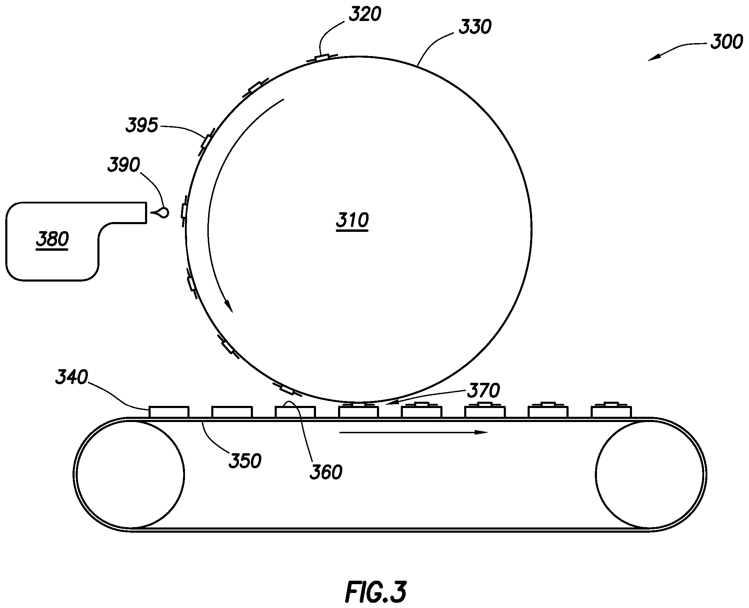

Another example of an assembly unit of the invention is shown in FIG. 3. In assembly unit 300 shown in FIG. 3, ingestible event marker identifier feeder 235 of the assembly unit 200 shown in FIG. 2 is replaced with rotating disc 310, which displays ingestible event marker identifiers 320 on its outer surface 330. Rotating disc 310 moves at a rate relative to the rate of pre-made tablets 340 on conveyer 350 in a manner such that an ingestible event marker identifier 320 is placed on the upper surface 360 of the pre-made tablet 340 at position 370. Also shown is an adhesive dispenser 380 which deposits a volume of adhesive 390 on the bottom surface 395 of the ingestible event marker identifiers 320 prior to contact of the bottom surface 395 of the ingestible event marker identifiers 320 with the upper surface 360 of the pre-made tablets 340.

The assembly units depicted in FIGS. 2 and 3 employ a physiologically acceptable adhesive, such as a thermoset or solvent-evaporation adhesive, in order to stably associate an ingestible event marker to a pre-made tablet carrier. However, other stable association approaches may be used by assembly units of the invention. For example, ingestible event marker identifiers and/or pre-made tablets may include one or more welding structures, such as tabs, which may be subjected to high energy (such as may be provided by a laser) in order to adhere the ingestible event marker identifier to the pre-made tablet. Such assembly units include sources of high energy, such a lasers, ultrasonic sources, etc. Alternatively, the assembly unit may include one or more pulse-jet devices, e.g., for assembling an ingestible event marker identifier on a surface of a pre-made tablet, as described in greater detail above.

Also of interest are instances where the ingestible event marker identifier is configured to provide for mechanical stable association with pre-made tablet upon assembly by the assembly unit. For example, the ingestible event marker identifier may be integrated with a snap-fit component, an elastomeric component such as a band or half-capsule, or analogously functioning structure that may be associated with the pre-made tablet by the assembly unit to provide mechanical stable association of the ingestible event marker identifier with the pre-made tablet. In some instances, a component of the ingestible event marker itself is configured to provide the mechanical stable association. For example, the signal amplification element, when present, may be configured to snap-fit around at least a portion of the pre-made tablet to provide for the desired stable association. In such instances, the signal amplification element may be fabricated from a material, such as a hydrogel material, that changes configuration upon contact with a fluid to provide for a desirable outcome, for example where the identifier is released from the carrier upon contact with the target physiological site.

Also of interest are assembly units that position an ingestible event marker identifier at an internal location of a pre-made tablet. Such assembly units may include a device for producing an indented structure, such as a hole, cavity or analogous structure, on a surface of the pre-made tablet, where the structure is configured to completely house an ingestible event marker identifier. Any convenient device for producing such a structure may be present in the assembly unit, where devices of interest include laser drilling devices, mechanical drills, etc. Once the indented structure is formed, the assembly unit may position an ingestible event marker in the indented structure. Any convenient placement device may be present in the assembly unit, such as the ingestible event marker identifier dispensers described above. In some instances, the ingestible event marker identifier may be configured to completely fill the indented structure, for example where the ingestible event marker identifier is configured to be press-fit into the indented structure. In other instances, the assembly unit may further include a device for filling any void space with a convenient medium, such as a solid or liquid sealing medium, that seals the ingestible event marker identifier in the indented structure. When the medium is a powder, the assembly unit may further include a press for compressing the power and thereby sealing the ingestible event marker identifier in the indented structure.

Assembly units of the invention may also be configured to work with tablet precursors to produce ingestible event markers.

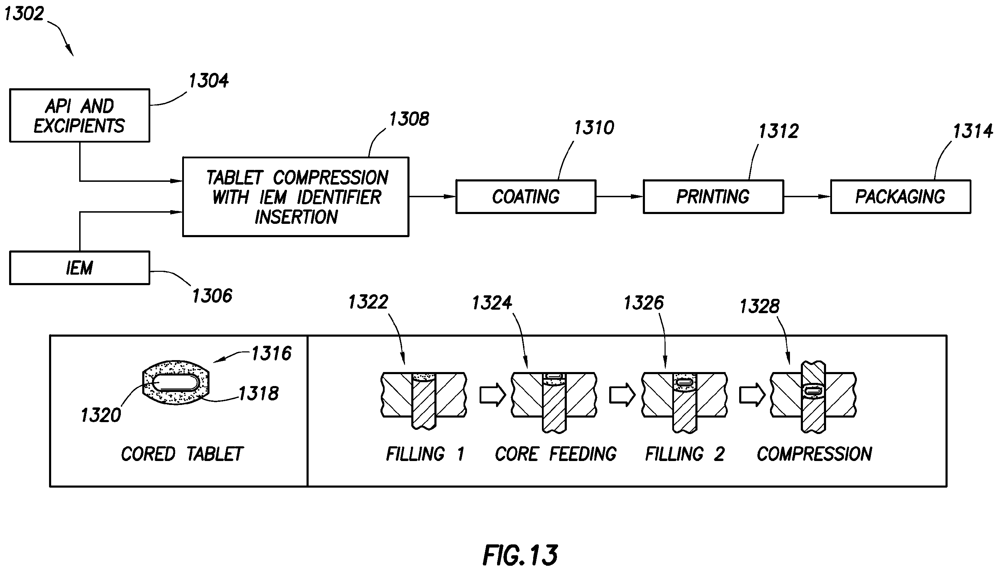

FIG. 13 provides a view of a process flow diagram 1302 for a system that combines an ingestible event marker (IEM) 1306 with an API and excipients 1304, e.g., table compression with IEM identifier insertion at step 1308 produces a product for coating at step 1310, printing at step 1312, and packaging at step 1314. Tablet precursors may be powders, one or more pre-made tablet components, etc., that are combined with the ingestible event marker identifier by the assembly unit to produce the product. In an example of the pre-made tablet components, a cored tablet 1316 having a tablet portion 1318 and a core 1320, and may be assembled by various processes, e.g., filling 1 at step 1322, core feeding at step 1324, filling 2 at step 1326, and core compression at step 1328.

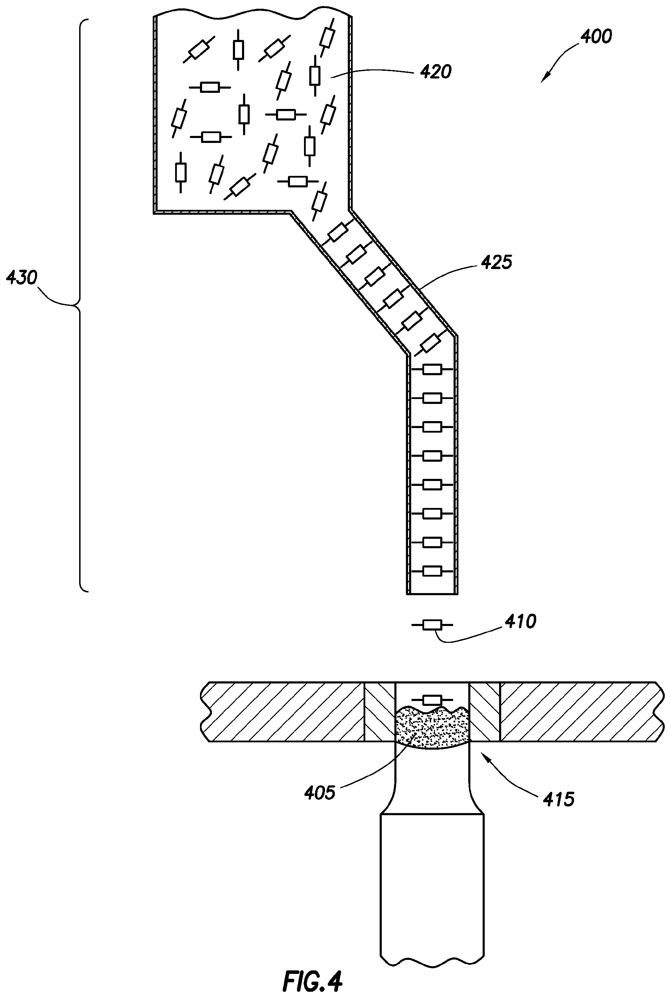

FIG. 4 provides a schematic of an assembly unit 400 configured to combine a powdered tablet precursor 405 composition with an ingestible event marker identifier 410 to produce an ingestible event marker. In FIG. 4, assembly unit 400 includes tablet die bottom half 415. Also shown is ingestible event marker identifier dispenser 430 which includes hopper 420 and feeder 425 which dispenses an ingestible event marker identifier 410 into the tablet die bottom half 415. A predetermined amount of tablet precursor powder 405 is present in tablet die bottom half 415. This tablet die precursor may have been positioned in the tablet die bottom half of 415 by any convenient protocol, such as by positioning the tablet die bottom half 415 in receiving relationship from a powder dispenser (not shown). The sequence of placement of tablet precursor powder and ingestible event marker identifier may vary, where in some instances the ingestible event marker identifier is placed in the tablet die bottom half 415 followed by the tablet precursor powder 405, and in other instances this order is reversed. In yet other instances, a first portion of the tablet precursor powder may be placed in the tablet die bottom half 415, followed by placement of the ingestible event marker identifier 410 and then placement of a second portion of the tablet precursor powder by moving the tablet die bottom half 415 into receiving relationship with a tablet powder dispenser (not shown). Following placement of the precursor powder 405 and ingestible event marker identifier 410 in the tablet die bottom half 415, the tablet die top half (not shown) is joined to tablet die bottom half 415 to apply sufficient pressure to produce the product tablet, which product tablet includes an ingestible event marker identifier and is therefore an ingestible event marker. Where desired, ingestible event marker identifiers having signal amplification elements configured to enhance association of the identifier with product tablet component may be employed. For example, signal amplification element may have holes or cutouts in it to allow the tablet to form around it (for example through the holes) so that the tablet does not split apart following production.

Tablet shapes and/or compositions may be employed which minimize stress on the ingestible event marker and produce more uniform stress distribution within a tablet that is produced. For example, tablet shapes having round or beveled edges may be produced.

In assembly units such as those shown in FIG. 4, the identifier is subjected to pressure during tablet formation. In such instances, the identifier may be associated with a protective component which serves to protect the integrity of the identifier during the tablet formation process. The protective component may vary, where examples of such components include coatings, such as those described above. In some instances the protective component envelopes the identifier and is fabricated from a physiologically acceptable material. The protective component in such instances may be pliable, so as to yield under application of pressure and therefore protect the identifier. Of interest in certain instances are gel materials.

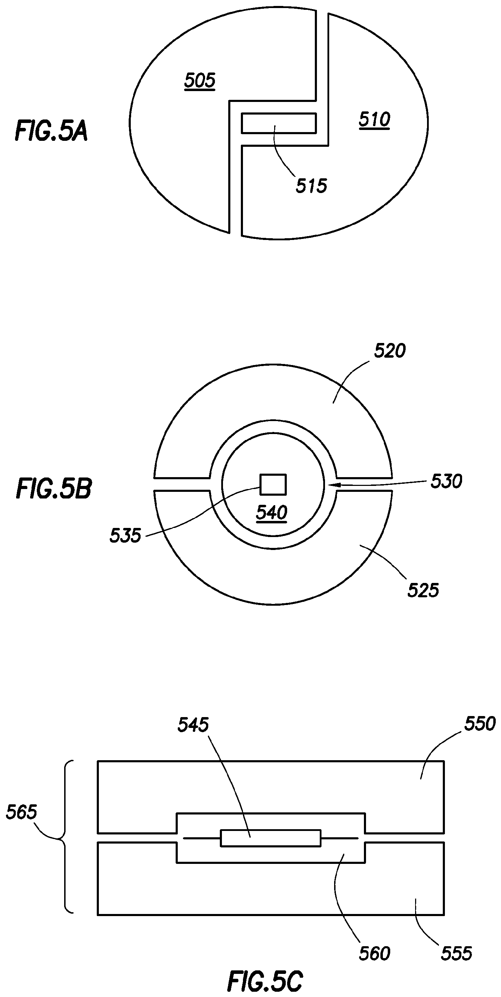

Instead of powders, tablet precursors may be two or more pre-formed tablet precursors which are combined with the ingestible event marker identifier in the assembly unit to produce the ingestible event marker. An example of how two tablet die precursors may be combined with an identifier is shown in FIG. 5A. During assembly, two separate feeders deliver to an assembly site of an assembly unit a pre-made half of a tablet, 505 and 510. The pre-made tablet halves 505 and 510 are configured to be combined with an ingestible event marker identifier 515 as shown to produce an ingestible event marker. An alternative configuration is shown in FIG. 5B. In FIG. 5B, pre-made tablet halves 520 and 525 are configured to be positioned about ingestible event marker identifier 530, where the identifier includes an integrated circuit component 535 and signal amplification element 540. Pre-made tablet precursors may also be upper and lower tablet halves 550 and 555 as shown in FIG. 5C. In producing an ingestible event marker from the tablet halves 550 and 555 shown in FIG. 5C, the assembly unit places identifier 545 into receiving space 560 of bottom tablet half 550 and then places top tablet half 555 over the upper surface of bottom tablet half 550 to produce the final ingestible event marker 565, in which the ingestible event marker identifier 545 is positioned inside of the tablet. In each of the above instances, the two pre-made tablet precursor halves may be secured to each other through use of a suitable adhesive, e.g., as described above.

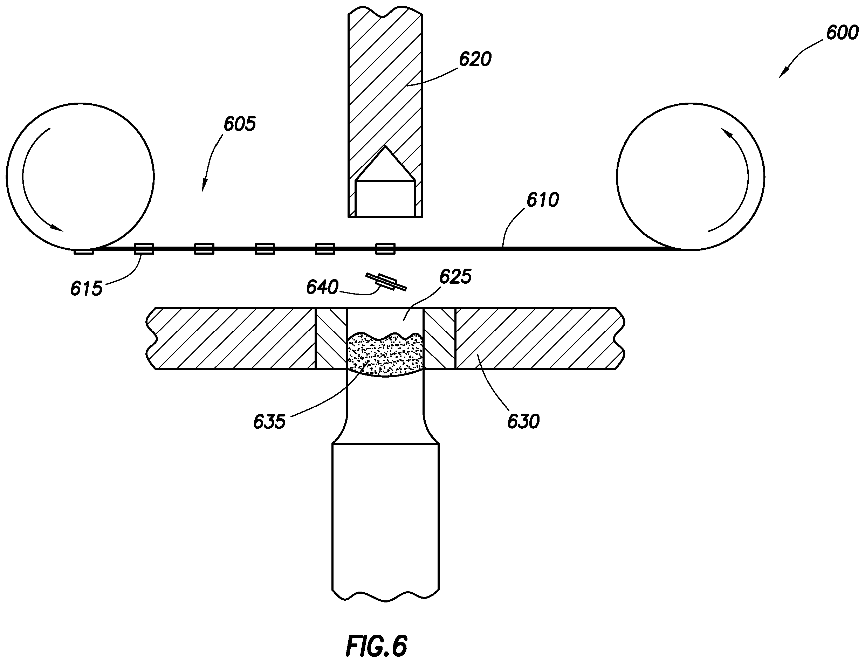

As summarized above, some systems of the invention employ punch elements in the assembly units. An example of such an assembly unit is shown in FIG. 6. In FIG. 6, assembly unit 600 includes reel-to-reel transfer device 605 on which an ingestible event marker precursor tape composition 610 is wound. Precursor tape composition 610 is made up of a tape fabricated from a material suitable for use as a signal amplification element of an ingestible event marker identifier. Positioned in equal spacing along the center axis of the tape 610 are ingestible event marker identifier circuitry components 615. When a circuitry component 615 is aligned with punch 620, punch 620 punches an ingestible event marker identifier 640 from the precursor tape. The resultant punched ingestible event marker identifier 640 falls into bottom half 625 of die press 630, which is filled with tablet precursor powder 635. Following placement of the ingestible event marker identifier 640 in the tablet die bottom half 625, the tablet die top half (not shown) is joined to tablet die bottom half 625 to apply sufficient pressure to produce the product tablet, which product tablet includes an ingestible event marker identifier and is therefore an ingestible event marker.

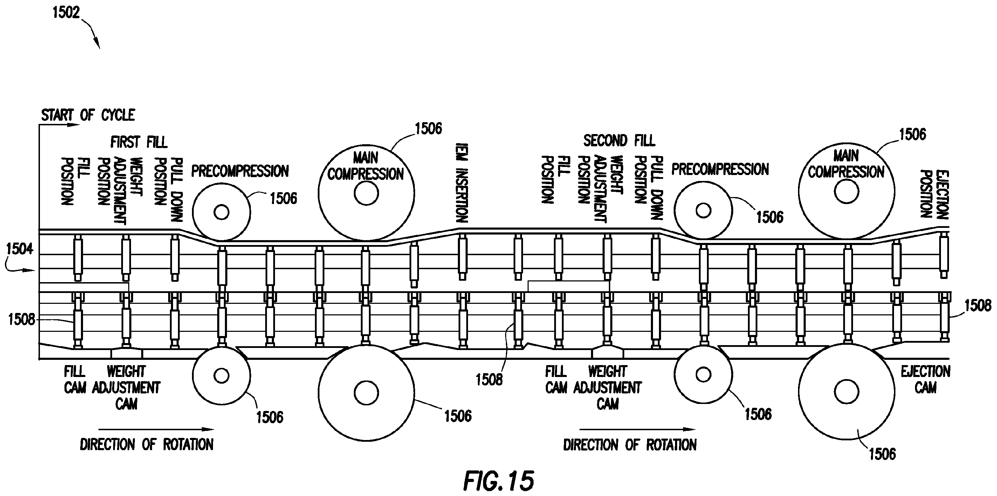

FIG. 15 provides a view of an automated ingestible event marker feed 1502 on a rotary tablet press system 1504. For example, at start of cycle, a first fill may begin with a fill position and fill cams 1508 moving though various phases, e.g., weight adjustment via weight adjustment cam; pre-compression via wheel 1506, main compression via wheel 1506. At second fill, the IEM insertion may occur at a fill position, then weight adjustment, pull down, recompression and ejection may occur.

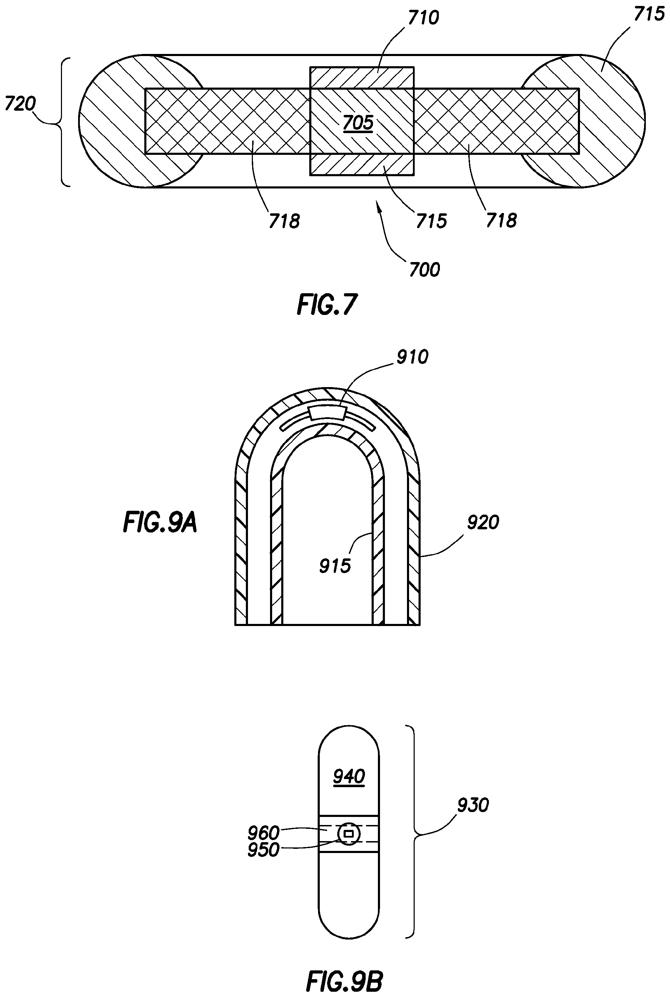

In some instances, tablets configurations in which the ingestible event marker identifier is accessible by a passageway or hole, for example to provide for ease of post-production inspection and/or programming, may be produced. For example, the assembly unit may produce a donut-shaped tablet as shown in FIG. 7 around a pre-made ingestible event marker identifier and a tablet precursor powder. FIG. 7 provides a cross-sectional view of ingestible event marker 720. In FIG. 7, ingestible event marker 720 is made up of ingestible event marker identifier 700 positioned inside of donut-shaped tablet 715. The ingestible event marker identifier 700 includes integrated circuitry component 705, upper and lower electrodes 710 and 715 and signal amplification element 718. To produce such ingestible event markers, the assembly unit may include a tablet press or mold configured to receive an ingestible event marker and tablet powder precursor and press the powder precursor about the circumference of the signal amplification element 718 to produce the end product. In a variation of such an assembly unit, the assembly unit may be configured to employ an ingestible event marker identifier precursor instead of a pre-made ingestible event marker identifier. For example, the assembly unit may combine an ingestible event marker identifier precursor that includes only the integrated circuitry component 705 and signal amplification element 718 with the powder precursor in the donut-shaped tablet press. Following pressing, the final components of the identifier, such as the upper and lower electrodes 710 and 715 may be produced, e.g., by using a pulse-jet device such as described above.

In some instances, tablet press systems currently known in the art are modified to have an ingestible event marker production unit as described above. Tablet press systems that may be so modified include, but are not limited to, those described in U.S. Patent Nos. 7,296,987; 7,060,294; 6,972,105; 5,958,467; 5,838,571.

When the tablet is pressed and where desired, the tablet may be embossed with a certain feature (such as an indent) to allow the produce ingestible event marker to be identified downstream. This way, tablets with multiple ingestible event marker identifiers can be readily identified.

With any tablet configuration produced by systems of the invention, it may be desirable to select tablet dimensions, such as tablet thicknesses, to ensure tablet disintegration and release of the identifier upon contact with the target physiological site, such as the stomach. In some instances, the tablet thickness ranges from 1 to 10 mm, such as 1 to 5 mm.

Instead of tablet configurations, ingestible event markers may have capsule configurations. In capsule configurations, first and second capsule components are configured to enclose an internal volume in which is located a filler composition, such as a pharmaceutically active agent composition or a placebo composition. This internal composition may be a liquid or solid, where solids of interest include powders, tablets, coated particulate compositions, pellets, beads and spherules. Filler compositions of capsules may vary. Filler compositions may include one or more vehicle components. Vehicle components may include one or more constituents, including but not limited to fillers, binders, disintegrants, coloring agents, etc. Vehicle components of interest are further reviewed in PCT Application Serial No. US2006/016370 published as WO 2006/116718, the disclosure of which is herein incorporated by reference. Additional disclosure of components that can be present in compositions of the invention can be found in Remington's Pharmaceutical Sciences, Mace Publishing Company, Philadelphia, Pa., 17th ed. (1985). Depending on the particular application for which the ingestible event marker is being fabricated, the ingestible event marker may not include a pharmaceutically active agent, e.g., as described above.

The first and second capsule components may be fabricated from any convenient material using any convenient protocol. Materials of interest from which the carrier components may be fabricated include physiologically acceptable polymeric materials that are used in conventional pharmaceutical capsule dosages. The materials may be clear or opaque, and may be colored as desired. Of interest are both rigid and elastic materials.

Suitable polymers from which carrier components of the invention may be fabricated include, but are not limited to: polyvinyl alcohol (PVA); natural and synthetic polysaccharides, including pullulan, carrageenan, xanthan, chitosan agar gums, and cellulosic materials, such as carboxymethylcellulose, hydroxypropylmethylcellulose (HPMC), methylcellulose, hydroxyethylcellulose, hydroxyethyl methylcellulose, hydroxypropylcellulose; polyethylene glycols (PEGs), polyethylene oxides (PEOs), mixtures of PEGs and PEOs; acrylic and methacrylic acid based polymers, such as EUDRAGIT E^{™}, EUDRAGIT L^{™} and/or EUDRAGIT S^{™} methacrylic acid polymers), EUDRAGIT RL^{™} and/or EUDRAGIT RS^{™} ammonium methacrylate copolymers; povidone (polyvinyl pyrrolidone), polyglycolysed glycerides (such as GELUCIRE 44/14^{™}, GELUCIRE 50/02^{™}, GELUCIRE 50/13^{™} and GELUCIRE 53/10^{™} polymers); carboxyvinyl polymers (such as CARBOPOL^{™} polymers); polyoxyethylene-polyoxypropylene copolymers (such as POLOXAMER188^{™} polymers); and the like.

The capsule components may be fabricated using any convenient protocol, including molding, etc. Fabrication protocols of interest include, but are not limited to, those described in U.S. Patent Nos.: 5,705,189; 4,576,284; 4,591,475; 4,655,840; 4,738,724; 4,738,817 and 4,790,881. Alternatively, the carrier component may be obtained from a commercial vendor, such as Qualicaps, Inc., Whitsett, NC.

In assembly units configured to make capsule-configured ingestible event markers, the assembly units may include transfer elements, such as feeds, conveyors, punches, rollers, etc., from sources of the components to an assembly site. In addition, a transfer element from a source of capsule filler composition (which may or may not include an active agent) to the assembly site may also present. The assembly unit may also include a mechanism to stably associate the one or more ingestible event marker identifiers, the first and second capsule components and the filler composition to produce a capsule ingestible event marker. FIG. 14 provides a view of process flow diagram 1402 for a system that combines an ingestible event marker with capsule components to produce an ingestible event marker. In process flow diagram 1402, an API and excipients 1404, e.g., capsule filling is provided and an IEM identifier 1406 is inserted into the API, e.g., capsule at step 1408. The capsule is branded and coated at step 1410 and packaged at step 1412.

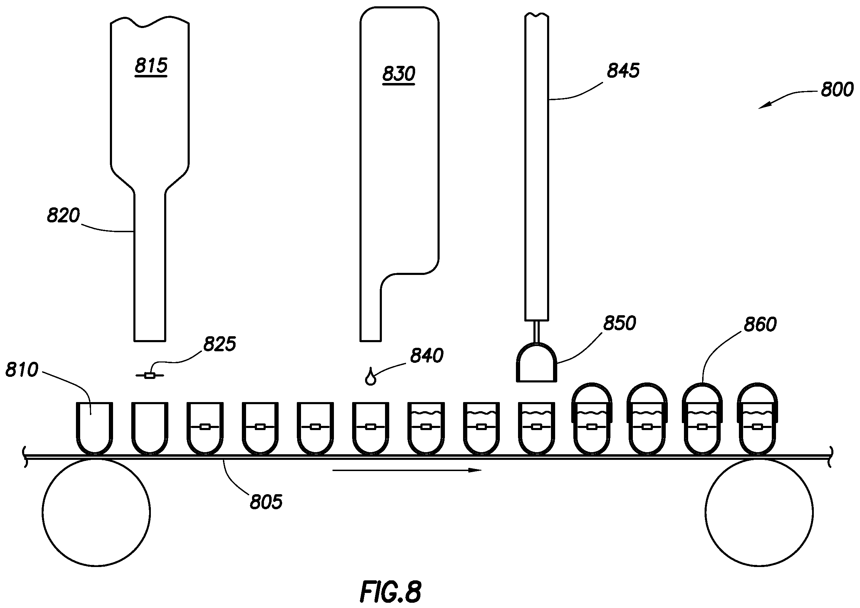

FIG. 8 provides a view of a capsule ingestible event marker assembly unit 800. In assembly unit 800, conveyer 805 moves bottom capsule halves 810 past the dispensing location of ingestible event marker identifier hopper 815 and feed 820, which dispenses an ingestible event marker identifier 825 into a capsule bottom half 810. Where desired, the ingestible event marker identifier 825 may be secured to the bottom of the bottom half 810, for example with a suitable adhesive and/or by press-fitting Dispenser 830 delivers a predetermined volume of liquid capsule filler composition 840 (which may or may not include a pharmaceutically active agent) into the bottom half 810. Robotic transfer element 845 then transfers top capsule half 850 from a source (not shown) to a sealing relationship with bottom half 810 to produce a product capsule 860 that includes the ingestible event marker identifier 825 in its internal volume. Because the ingestible event marker identifier 825 is present in the capsule 860, the capsule 860 is an ingestible event marker. As with the tablet production systems described above, various components of the system 800 shown in FIG. 8 may be modified. For example, the ingestible event marker identifier hopper 815 and feed 820 may be replaced with a punch component, e.g., as shown in FIG. 6 or a dispenser as shown in FIG. 2 (which dispenser may be an air gun.)

In a variation of the assembly unit shown in FIG. 8, the assembly unit 800 may be modified to replace the top capsule halves as shown with ingestible event marker caps as described in co-pending U.S. provisional patent application serial no. 61/142,861 and titled, "Pharmaceutical Dosages that Include an Ingestible Event Marker Identifier" the entire disclosure of which is incorporated herein by reference. In these instances, a separate source of ingestible event marker identifiers may not be present.

In yet another variation of the assembly unit shown in FIG. 8, capsules having ingestible event markers integrated into one of the capsule halves are employed, such that a separate source of ingestible event marker identifiers is not necessary. Ingestible event marker identifiers may be incorporated into capsule halves using any convenient approach. Examples of suitable approaches for producing capsule halves that include integrated ingestible event markers are provided in United States Application Serial No. 12/238,345, the disclosure of which is herein incorporated by reference.

In yet other aspects, the assembly unit may be configured to stably associate an ingestible event marker with a capsule carrier by sandwiching an ingestible event marker identifier between two capsule halves. For example, FIG. 9A shows an ingestible event marker identifier 910 sandwiched between first capsule half 915 and second capsule half 920, where first capsule half 915 is configured to fit inside of second capsule half 920 as shown.

Alternatively, the assembly unit may be configured to stably associate an ingestible event marker identifier with a capsule half by employing a physiologically acceptable adhesive to adhere the ingestible event marker identifier with a surface of the capsule half, such as an outer surface of the capsule half. The resultant capsule may then be filled and sealed, as desired to produce the final ingestible event marker. A variation of the assembly unit shown in FIG. 2, where the pre-made tablets are replaced with capsule halves and then the capsule halves are filled and capped as shown in FIG. 8 may be employed to assemble this configuration of the ingestible event marker.

In yet another type of capsule ingestible event marker, the ingestible event marker may be mechanically stably associated with the capsule where the ingestible event marker identifier is associated with a structure that provides for this mechanical stable association. FIG. 9B provides an example of such a capsule ingestible event marker. In FIG. 9B, ingestible event marker identifier 930 includes pre-made capsule 940 and ingestible event marker identifier 950 secured to the outer surface thereof by elastic band 960. To fabricate this structure, an assembly unit may employ pre-made capsules 940 and ingestible event marker identifiers integrated with elastic bands (950 and 960), where the assembly unit is configured to slide the elastic band 960 over the capsule 940 to produce the ingestible event marker 930.

In yet other instances, the assembly unit is configured to inject an ingestible event marker identifier into the interior of a pre-made capsule. For example, the assembly unit may include an air gun or analogous device that injects an ingestible event marker identifier into the interior of a pre-made capsule. In such instances, the assembly unit may further be configured to seal any resultant hole that results from the injection.

In some instances, capsule manufacturing systems currently known in the art are modified to have an ingestible event marker production unit as described above. Capsule press systems that may be so modified include, but are not limited to, those described in U.S. Patent Nos. 7,082,738; 7,025,911; 6,877,611; 6,499,279; 6,245,350; 6,080,426; 5,765,342.

In some instances, the assembly unit is integrated with a packaging unit of the system. For example, the system may be configured to produce blister packages of the ingestible event markers. In such systems, assembly components configured to stably associate an ingestible event marker with a carrier, such as a tablet or capsule, may be integrated with the blister-packaging unit of the system. An example of such a system is a system where the blister-packing unit includes one or more devices for assembling an ingestible event marker identifier on a surface of a pre-made carrier, such as a capsule or tablet. Examples of ingestible event marker identifier assembly devices that may be integrated with the packaging unit include pulse-jet deposition devices, which deposit controlled amounts of material onto precise locations in a manner sufficient to produce an ingestible event marker.

High-throughput ingestible event marker production systems of the invention may include additional units to perform other functions in the assembly of ingestible event markers. Upstream of the ingestible event marker assembly unit may be units configured to make components of the ingestible event markers, such as ingestible event marker identifiers, pre-made tablets, tablet precursors, capsule halves, capsule filler compositions, pre-made tablets, etc. Downstream of the ingestible event marker assembly units may be one or more subsequent units, such as counter units, sorting units, packaging units and the like.

Also present in systems of the invention may be one or more quality control elements, such as cameras, which check the quality of the ingestible event markers produced by the systems and reject those markers that do not meet one or more pre-determined quality thresholds, such as but not limited to shape, color, size, etc. Examples of quality control elements of interest that may be present in systems of the invention include, but are not limited to those described in United States Patent Nos. 6,741,731; 6,739,455; 6,434,911; 6,079,284; 5,522,512.

In some instances, the quality control unit includes an ingestible event marker identifier detector configured to specifically detect ingestible event marker identifiers associated with carriers in an ingestible event marker. One type of ingestible event marker identifier detector of interest is a metal detector configured to detect the presence of ingestible event marker identifiers in the final ingestible event markers (where the ingestible event marker identifiers will include metals that will be detected by the metal detector). Other types of ingestible event marker identifier detectors of interest that may be included in the quality control units of the systems include, but are not limited to: X-ray based detectors, near infra-red based detectors, etc.

Also of interest are quality control units that include operational detectors configured to confirm that the ingestible event marker identifiers of the ingestible event markers produced by the system are operational. For example, quality control units may include radio frequency (RF) devices, such as near-field RF devices, configured to confirm that the integrated circuit component of the ingestible event marker is operational. Alternatively, the quality control unit may be configured to print traces on a carrier of a product ingestible event marker (for example by pulse-jet techniques) and then use the printed traces to provide connection to a probing device that checks the operability of the circuitry of the ingestible event marker identifier.

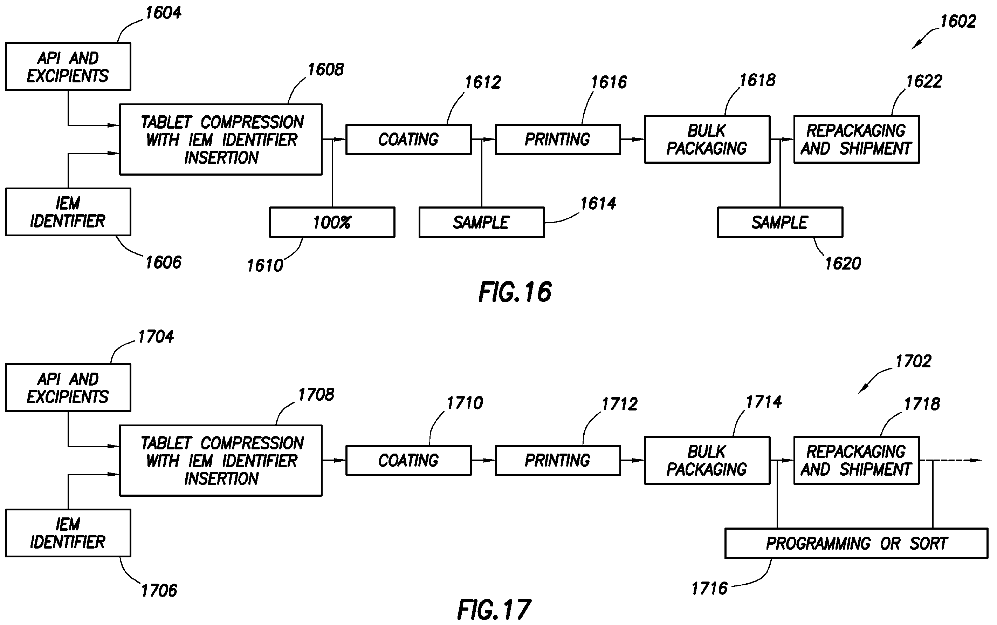

A process flow diagram 1602 of a system configured to inspect ingestible event markers following production is shown in FIG. 16. In the process flow diagram 1602, an API and excipients 1506, e.g., tablet components, may be compressed and an IEM identifier 1606 inserted at step 1608 at 100% 1510; a coating may be applied at 1612 and sampled at step 1614. Printing may be accomplished at step 1616 and the products bulk-packaged at step 1618, then sampled at 1620, and the product repackaged and shipped at step 1622.

In some instances, systems of the invention may include post-production programming devices that are configured to program the circuitry components of the ingestible event marker identifiers of the product ingestible event markers. Any convenient programming devices may be employed. For example, the operational detectors described above may further include programming functionality which programs the identifier before, during and/or after operational determination. Also of interest are electrostatic programming devices.

A process flow diagram 1702 of a system configured to program ingestible event markers following production is shown in FIG. 17. In the process flow diagram 1702, an API and excipients 1604, e.g., tablet components may be compressed and an IEM identifier 1706 inserted at step 1708 to produce a product. The product may be coated at step 1710, printed at step 172, bulk-packaged at step 1714, programming or sorting associated with the products may be accomplished at step 1716 and repackaging and shipment of the product may be accomplished at step 1718.

In some instances, the systems of the invention are further configured to associate a signature element with the ingestible event marker that uniquely identifies the ingestible event marker identifier of the ingestible event marker and can be read at any convenient time, such as the time of sale, to ensure authenticity of the ingestible event marker (for example to detect any counterfeiting activity that may have occurred in the supply chain between manufacture and the point of sale), e.g., via a signature element unit. This signature element may be any convenient collection of symbols, numbers or letters or combinations thereof, as desired. The signature element may be readily human or machine readable or encrypted, as desired. The signature element may be associated with the ingestible event marker in any convenient way, such as printed onto the ingestible event marker, or printed onto packaging associated therewith, such as a blister pack, bottle, box, etc.

Systems of the invention may conveniently be under computer control, where any suitable architecture may be employed. Of interest are computer control units that include distributed control architectures, for example where the architecture is designed to optimize data flow between the distributed control elements of the architecture. An example of such control architecture may include a controlling supervisor computer which communicates via serial interfaces with a number of custom interface boards associated with different units of the system, each unit with its own computer processing unit (CPU). For example, a computer control unit may include a controlling supervisor computer in communication with a CPU associated with each of the various units of a system of the invention, including an assembly unit as well as other units that may be present, such as but not limited to, precursor fabrication units, sorting units, quality control units, packaging units, etc. The computer control system may include an operator terminal including a video display unit displaying information pertinent to a user, as desired. Examples of computer control units of interest that may be adapted to operate systems of the invention include, but are not limited to those described in United States Patent Nos. 7,293,672; 7,082,738; 6,816,793; 6,499,279; 5,522,512.

Systems of the invention may be configured to run on any convenient power source or combination of power sources. As such, systems of the invention may be powered pneumatically, electrically, or via combinations thereof.

It is to be understood that this invention is not limited to particular aspects described, as such may vary. It is also to be understood that the terminology used herein is for the purpose of describing particular aspects only, and is not intended to be limiting, since the scope of the present invention will be limited only by the appended claims.

Where a range of values is provided, it is understood that each intervening value, to the tenth of the unit of the lower limit unless the context clearly dictates otherwise, between the upper and lower limit of that range and any other stated or intervening value in that stated range, is encompassed within the invention. The upper and lower limits of these smaller ranges may independently be included in the smaller ranges and are also encompassed within the invention, subject to any specifically excluded limit in the stated range. Where the stated range includes one or both of the limits, ranges excluding either or both of those included limits are also included in the invention.

Unless defined otherwise, all technical and scientific terms used herein have the same meaning as commonly understood by one of ordinary skill in the art to which this invention belongs. Although any methods and materials similar or equivalent to those described herein can also be used in the practice or testing of the present invention, representative illustrative methods and materials are now described.

All publications and patents cited in this specification are herein incorporated by reference as if each individual publication or patent were specifically and individually indicated to be incorporated by reference and are incorporated herein by reference to disclose and describe the methods and/or materials in connection with which the publications are cited. The citation of any publication is for its disclosure prior to the filing date and should not be construed as an admission that the present invention is not entitled to antedate such publication by virtue of prior invention. Further, the dates of publication provided may be different from the actual publication dates which may need to be independently confirmed.

It is noted that, as used herein and in the appended claims, the singular forms "a", "an", and "the" include plural referents unless the context clearly dictates otherwise. It is further noted that the claims may be drafted to exclude any optional element. As such, this statement is intended to serve as antecedent basis for use of such exclusive terminology as "solely," "only" and the like in connection with the recitation of claim elements, or use of a "negative" limitation.

Certain ranges have been presented herein with numerical values being preceded by the term "about." The term "about" is used herein to provide literal support for the exact number that it precedes, as well as a number that is near to or approximately the number that the term precedes. In determining whether a number is near to or approximately a specifically recited number, the near or approximating unrecited number may be a number which, in the context in which it is presented, provides the substantial equivalent of the specifically recited number.

As will be apparent to those of skill in the art upon reading this disclosure, each of the individual aspects described and illustrated herein has discrete components and features which may be readily separated from or combined with the features of any of the other several aspects without departing from the scope or spirit of the present invention. Any recited method can be carried out in the order of events recited or in any other order which is logically possible.

Although the foregoing invention has been described in some detail by way of illustration and example for purposes of clarity of understanding, it is readily apparent to those of ordinary skill in the art in light of the teachings of this invention that certain changes and modifications may be made thereto without departing from the spirit or scope of the appended claims.

### CLAUSES

The following clauses 1 to 15 reproduce the complete text of the Clauses as originally filed in the parent application EP10729424.1 (PCT/US2010/020142). They are included herein in order to preserve the whole content of the parent application for the purpose of supporting possible future amendments of the present application and/or any further divisional application. They are not to be regarded as Clauses within the meaning of Art. 78(1)(c) EPC.
1. A high-throughput system comprising an automated assembly unit configured to stably associate an ingestible event marker with a carrier to produce a product.
2. The system according to Clause 1, wherein the assembly unit is configured to stably associate an ingestible event marker with a pre-made carrier.
3. The system according to Clause 2, wherein the assembly unit is configured to stably associate the ingestible event marker with the pre-made carrier using an adhesive.
4. The system according to Clause 2, wherein the ingestible event marker includes an element that mechanically provides for stable association with the pre-made carrier.
5. The system according to Clause 2, wherein the assembly unit is configured to print one or more components of the ingestible event marker on a surface of the pre-made carrier.
6. The system according to Clause 1, wherein the assembly unit is configured to produce a product from an ingestible event marker and a carrier precursor.
7. The system according to Clause 1, wherein the assembly unit is configured to associate a coating with the ingestible event marker.
8. The system according to Clause 7, wherein the coating is opaque and is configured to hide the ingestible event marker associated with the carrier.
9. The system according to Clause 1, wherein the assembly unit is integrated with a packaging unit of the system.
10. The system according to Clause 1, wherein the system comprises an element selected from the group consisting essentially of:
   an ingestible event marker manufacturing unit to manufacture ingestible event marker;
   a quality control inspection unit to inspect the ingestible event marker;
   a programming device to program the ingestible event marker of the product;
   a signature element unit to associate a signature with the ingestible event marker; and
   a packaging unit to produce packages associated with the ingestible event markers.
11. A high-throughput method for producing a product, the method comprising: assembling an ingestible event marker with an assembly unit configured to stably associate an ingestible event marker with a carrier to produce the product.
12. The method according to Clause 11, wherein the assembling an ingestible event marker with an assembly unit configured to stably associate an ingestible event marker with a carrier to produce a product comprises: compressing a carrier with the ingestible event marker to form the product.
13. The method according to Clause 11, further comprising: coating at least a portion of the ingestible event marker.
14. The method according to Clause 11, further comprising: printing to deposit one or more components of the ingestible event marker onto at least a portion of the carrier.
15. The method according to Clause 11, further comprising: packaging the product.
16. The method according to Clause 15, wherein the packaging the ingestible event marker further comprises: bulk packaging multiple products.
17. The method according to Clam 16, further comprising: repackaging the bulk-packaged products.
18. The method according to Clause 11, wherein the assembling an ingestible event marker with an assembly unit configured to stably associate an ingestible event marker identifier with a carrier to produce an ingestible event marker comprises: mechanically attaching the ingestible event marker to the carrier.
19. The method according to Clause 18, further comprising: overcoating at least a portion of the ingestible event marker.
20. The method according to Clause 11, wherein the assembling an ingestible event marker with an assembly unit configured to stably associate an ingestible event marker with a carrier to produce an ingestible event marker comprises: inserting an ingestible event marker into a carrier.
21. A process for manufacturing an identifiable product that includes a pharmaceutical agent and an ingestible device, the process comprising the step of presenting the pharmaceutical agent in the form of a tablet to an assembly unit, wherein the assembly unit manufactures the identifiable product through the steps comprising:
   placing a determined volume of adhesive onto one surface of the tablet to produce an adhesive sided tablet using an adhesive delivery unit; and
   securing the ingestible device to the adhesive sided tablet to produce the identifiable product using a device delivery unit.
22. The process of Clause 21, wherein the assembly unit further performs a step including placing a protective coating onto the identifiable product to produce a coated identifiable product using a protective coating delivery unit.
23. A process for manufacturing an identifiable product from a pharmaceutical agent and an ingestible device using an assembly unit that includes a powder delivery unit, a compressing unit, and a device delivery unit, the process comprising the steps of:
   delivering a first portion powder form of the pharmaceutical agent to a tablet die of the assembly unit using the powder delivery unit;
   placing the ingestible device into the tablet die of the assembly unit using the device delivery unit;
   delivering a second portion powder form of the pharmaceutical agent to the tablet die of the assembly unit; and
   compressing the content of the tablet die using the compressing unit to form the identifiable product.
24. A process for manufacturing an identifiable product using an assembly apparatus that includes a first capsule portion delivery unit, a capsule assembler unit, an agent delivery unit, and a device delivery unit, the process comprising the steps of:
   delivering a first portion of a capsule using the first capsule portion delivery unit of the assembly apparatus;
   filling the first portion of the capsule with a pharmaceutical agent using the agent delivery unit of the assembly apparatus;
   placing an ingestible device into the first portion of the capsule using the device delivery unit of the assembly apparatus; and
   sealing the content of the first portion of the capsule with a second portion of the capsule using the capsule assembler unit of the assembly apparatus.

## Claims

1. A high-throughput system comprising an automated assembly unit configured to stably associate an ingestible event marker with a carrier to produce a product, wherein the carrier preferably is a pre-made carrier and/or wherein the event marker is associated with the pre-made carrier using an adhesive and/or wherein the ingestible event marker includes an element that mechanically provides for stable association with the pre-made carrier and/or wherein the assembly unit is configured to print one or more components of the ingestible event marker on a surface of the pre-made carrier.

2. The system according to claim 1, wherein the assembly unit is configured to produce a product from an ingestible event marker and a carrier precursor.

3. The system according to claim 1 or 2, wherein the assembly unit is configured to associate a coating with the ingestible event marker, the coating preferably being opaque and preferably being configured to hide the ingestible event marker associated with the carrier.

4. The system according to any of claims 1-3, wherein the assembly unit is integrated with a packaging unit of the system.

5. The system according to any of the preceding claims, wherein the system comprises an element selected from the group consisting essentially of:
an ingestible event marker manufacturing unit to manufacture ingestible event marker;
a quality control inspection unit to inspect the ingestible event marker;
a programming device to program the ingestible event marker of the product;
a signature element unit to associate a signature with the ingestible event marker; and
a packaging unit to produce packages associated with the ingestible event markers.

6. A high-throughput method for producing a product, the method comprising:
assembling an ingestible event marker with an assembly unit, preferably an assembly unit in a system as defined in any of the preceding claims, the assembly unit being configured to stably associate an ingestible event marker with a carrier to produce the product.

7. The method according to claim 6, wherein the assembling an ingestible event marker with an assembly unit configured to stably associate an ingestible event marker with a carrier to produce a product comprises:
compressing a carrier with the ingestible event marker to form the product, or mechanically attaching the ingestible event marker to the carrier.

8. The method according to claim 5 or 6, further comprising:
coating at least a portion of the ingestible event marker.

9. The method according to any of the claims 5-8, further comprising:
printing to deposit one or more components of the ingestible event marker onto at least a portion of the carrier.

10. The method according to any of claims 5-9, further comprising:
packaging the product, wherein packaging the ingestible event marker preferably comprises bulk packaging multiple products and/or repackaging the bulk-packaged products.

11. The method according to any of claims 5-10, further comprising:
overcoating at least a portion of the ingestible event marker.

12. The method according to any of claims 5-11, wherein the assembling an ingestible event marker with an assembly unit configured to stably associate an ingestible event marker with a carrier to produce an ingestible event marker comprises:
inserting an ingestible event marker into a carrier.

13. A process for manufacturing an identifiable product that includes a pharmaceutical agent and an ingestible device, the process comprising the step of presenting the pharmaceutical agent in the form of a tablet to an assembly unit, preferably an assembly unit in a system as defined in any of claims 1-4, wherein the assembly unit manufactures the identifiable product through the steps comprising:
placing a determined volume of adhesive onto one
surface of the tablet to produce an adhesive sided tablet using an adhesive delivery unit; and
securing the ingestible device to the adhesive sided tablet to produce the identifiable product using a device delivery unit,
wherein the assembly unit preferably performs a step including placing a protective coating onto the identifiable product to produce a coated identifiable product using a protective coating delivery unit.

14. A process for manufacturing an identifiable product from a pharmaceutical agent and an ingestible device using an assembly unit that includes a powder delivery unit, a compressing unit, and a device delivery unit, preferably an assembly unit in a system as defined in any of claims 1-4, the process comprising the steps of:
delivering a first portion powder form of the pharmaceutical agent to a tablet die of the assembly unit using the powder delivery unit;
placing the ingestible device into the tablet die of the assembly unit using the device delivery unit;
delivering a second portion powder form of the pharmaceutical agent to the tablet die of the assembly unit; and
compressing the content of the tablet die using the compressing unit to form the identifiable product.

15. A process for manufacturing an identifiable product using an assembly apparatus that includes a first capsule portion delivery unit, a capsule assembler unit, an agent delivery unit, and a device delivery unit, preferably an assembly unit in a system as defined in any of claims 1-4, the process comprising the steps of:
delivering a first portion of a capsule using the first capsule portion delivery unit of the assembly apparatus;
filling the first portion of the capsule with a pharmaceutical agent using the agent delivery unit of the assembly apparatus;
placing an ingestible device into the first portion of the capsule using the device delivery unit of the assembly apparatus; and
sealing the content of the first portion of the capsule with a second portion of the capsule using the capsule assembler unit of the assembly apparatus.
